# EUROPEAN PATENT SPECIFICATION

(11) **EP 3 440 759 B1**
(45) Date of publication and mention of the grant of the patent: **10.06.2020**
(21) Application number: 17708945.5
(22) Date of filing: 21.02.2017
(51) Int. Cl.: A61B 5/00, H02J 7/02, A61N 1/375, A61N 1/378, A61N 1/40, A61N 1/37, H04B 5/00, H02J 50/12, H02J 50/27, H02J 50/23

(54) **APPARATUS AND METHOD FOR RECEIVING WIRELESS POWER AND COMMUNICATIONS**
VORRICHTUNG UND VERFAHREN ZUM EMPFANG VON DRAHTLOSER LEISTUNG UND KOMMUNIKATION
APPAREIL ET MÉTHODE POUR RECEVOIR DE L' ÉNERGIE SANS FIL ET DES COMMUNICATIONS

(30) Priority: 29.04.2016 US 201662329976 P; 17.02.2017 US 201715436601
(43) Date of publication of application: 13.02.2019
(73) Proprietor: Qualcomm Incorporated, San Diego, CA 92121-1714 (US)
(72) Inventor: JEONG, Seong Heon, San Diego, California 92121-1714 (US)
(74) Representative: Tomkins & Co
(86) International application number: PCT/US2017/018691
(87) International publication number: WO 2017/189079

(56) References cited:
- WO-A1-2016/028010
- US-A1- 2010 149 042
- US-A1- 2011 112 610
- US-A1- 2015 066 113
- XIAOYU CHENG ET AL: "An Omnidirectional Wrappable Compact Patch Antenna for Wireless Endoscope Applications", IEEE ANTENNAS AND WIRELESS PROPAGATION LETTERS, IEEE, PISCATAWAY, NJ, US, vol. 11, 1 January 2012 (2012-01-01), pages 1667-1670, XP011492811, ISSN: 1536-1225, DOI: 10.1109/LAWP.2013.2238600

## Description

### BACKGROUND

### Field

The present disclosure relates generally to wireless power transfer, and more specifically to methods and apparatus for wirelessly conveying power to electronic devices that may be implanted within or worn on a user body.

### Description of the Related Art

Electronic devices implanted within or worn on a user body may be damaged by exposure to various electrical signals or fields. In wireless power applications, wireless power charging systems may provide the ability to charge and/or power electronic devices without physical, electrical connections, thus reducing the number of components required for operation of the electronic devices and simplifying the use of the electronic device. Such wireless power charging systems may comprise a wireless power transmitter and other transmitting circuitry configured to generate a magnetic field that may be used to wirelessly transfer power to wireless power receivers. Accordingly, there is a need for methods and apparatus for protecting internal components from damage while receiving wireless power and/or data transmissions by receivers, for example receivers in medical implants or user worn medical devices.

US 2011112610 A1 discloses an improved implantable pulse generator (IPG) containing improved telemetry circuitry. The IPG includes charging and telemetry coils within the IPG case, which increases their mutual inductance and potential to interfere with each other. The improved telemetry circuitry includes decoupling circuitry for decoupling the charging coil during periods of telemetry between the IPG and an external controller. Such decoupling circuitry can comprise use of pre-existing LSK circuitry during telemetry, or new discrete circuitry dedicated to decoupling. The decoupling circuitry is designed to prevent or at least reduce induced current flowing through the charging coil during data telemetry. The decoupling circuitry can be controlled by the microcontroller in the IPG, or can automatically decouple the charging coil at appropriate times to mitigate an induced current without instruction from the microcontroller.

US 2015066113 A1 discloses an improved case for an implantable medical device having an internal communication coil in which a lower-conductivity, more-radio lucent metallic plate is provided proximate to the coil. The remainder of the case can be formed of a higher-conductivity metallic material which is easier to form and thus lends itself to the manufacture of implantable medical devices with smaller cases for example. As both the plate and the remainder of the case are metallic, they can be easily joined by reliable laser welding techniques for example.

WO 2016/028010 A1 discloses a wireless power reception device including an inner coil, a mounting member having the inner coil mounted thereon and a slit formed in an edge of the inner coil, and a first shielding member and a second shielding member laminated on the first shielding member and provided in correspondence with the inner coil, the mounting member being mounted on the first shielding member and the second shielding member. Accordingly, it is possible to improve efficiency of the wireless power reception device.

### SUMMARY

The present invention relates to an apparatus and a method as defined in the appended claims. Various implementations of methods and devices within the scope of the appended claims each have several aspects, no single one of which is solely responsible for the desirable attributes described herein. Without limiting the scope of the appended claims, some prominent features are described herein.

An aspect of this disclosure is an apparatus for receiving power wirelessly. The apparatus comprises a receive circuit configured to receive wireless communication and charging power. The apparatus also comprises a metallic structure defining a gap extending from a first surface to a second surface, and through the metallic structure, the first surface opposite the second surface. The metallic structure is configured to receive the charging power from a wireless charging field oscillating at a first frequency. The metallic structure is further configured to convey the received power to the receive circuit via first and second connecting feeds. The metallic structure is also configured to shield the receive circuit from interference at frequencies other than the first frequency.

### BRIEF DESCRIPTION OF THE DRAWINGS

Details of one or more implementations of the subject matter described in this specification are set forth in the accompanying drawings and the description below. Other features, aspects, and advantages will become apparent from the description, the drawings, and the claims.
FIG. 1 is a functional block diagram of a wireless power transfer system, in accordance with one exemplary implementation.
FIG. 2 is a functional block diagram of a wireless power transfer system, in accordance with another exemplary implementation.
FIG. 3 is a schematic diagram of a portion of transmit circuitry or receive circuitry of FIG. 2 including a transmit or receive antenna, in accordance with exemplary implementations.
FIG. 4 is a simplified functional block diagram of a transmitter that may be used in an inductive power transfer system, in accordance with exemplary implementations of the present disclosure.
FIG. 5 is a simplified functional block diagram of a receiver that may be used in the inductive power transfer system, in accordance with exemplary implementations of the present disclosure.
FIG. 6 shows a view of a wireless power transfer system 600 as applied to an area of a human body.
FIG. 7 shows a rendered schematic of a shield placed around electrical components of an implant.
FIG. 8A shows a first view of an implant having a shield and a two wire feed.
FIG. 8B shows a second view of the implant of FIG. 8A.
FIGs. 9A-9C show alternate configurations of the shield, slot, and/or bridge of the implant of FIGs. 7 and 8.
FIG. 10A shows a 3D graph corresponding to a radiation pattern of the antenna of the implant (e.g., the implant of FIG. 7).
FIG. 10B shows a graph indicating signal strength of the antenna of the implant (e.g., the implant of FIG. 7) as a function of frequency.
FIG. 11 is a flowchart that includes a plurality of steps of a method receiving wireless power and communication in an implantable device, in accordance with exemplary implementations of the present disclosure.

The various features illustrated in the drawings may not be drawn to scale. Accordingly, the dimensions of the various features may be arbitrarily expanded or reduced for clarity. In addition, some of the drawings may not depict all of the components of a given system, method or device. Finally, like reference numerals may be used to denote like features throughout the specification and figures.

### DETAILED DESCRIPTION

The detailed description set forth below in connection with the appended drawings is intended as a description of exemplary implementations and is not intended to represent the only implementations in which the present disclosure may be practiced. The term "exemplary" used throughout this description means "serving as an example, instance, or illustration," and should not necessarily be construed as preferred or advantageous over other exemplary implementations. The detailed description includes specified details for the purpose of providing a thorough understanding of the exemplary implementations. In some instances, some devices are shown in block diagram form.

Wirelessly transferring power may refer to transferring any form of energy associated with electric fields, magnetic fields, electromagnetic fields, or otherwise from a transmitter to a receiver without the use of physical electrical conductors (e.g., power may be transferred through free space). The power output into a wireless field (e.g., a magnetic field) may be received, captured by, or coupled by a "receiving coil" to achieve power transfer.

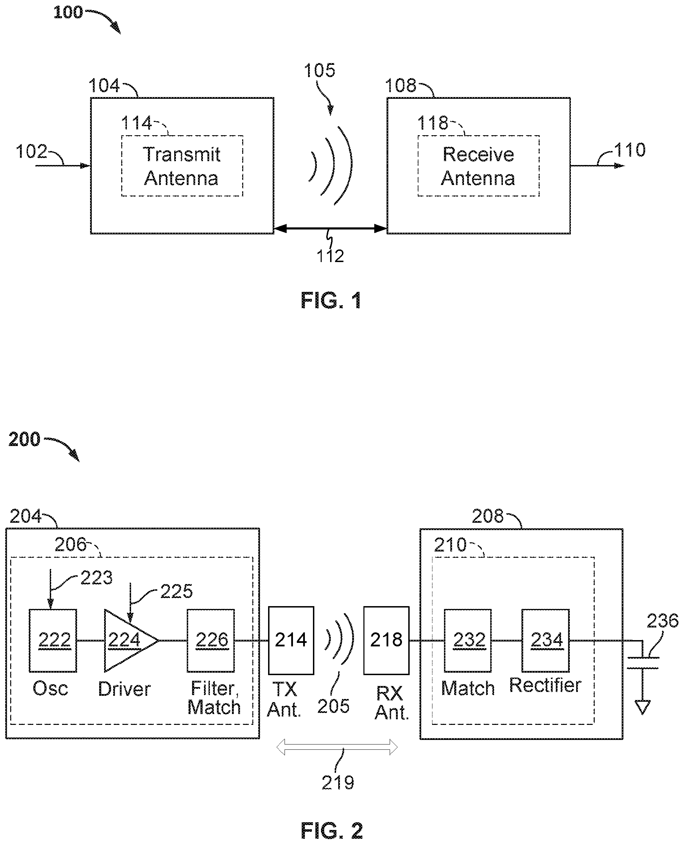

FIG. 1 is a functional block diagram of a wireless power transfer system 100, in accordance with one exemplary implementation. Input power 102 may be provided to a transmitter 104 from a power source (not shown) to generate a wireless (e.g., magnetic or electromagnetic) field 105 for performing wireless power transfer. A receiver 108 may couple to the wireless field 105 and generate output power 110 for storage or consumption by a device (not shown) coupled to the output power 110. Both the transmitter 104 and the receiver 108 are separated by a distance 112.

In one exemplary implementation, the transmitter 104 and the receiver 108 are configured according to a mutual resonant relationship. When the resonant frequency of the receiver 108 and the resonant frequency of the transmitter 104 are substantially the same or very close, transmission losses between the transmitter 104 and the receiver 108 are reduced. As such, wireless power transfer may be provided over a larger distance in contrast to purely inductive solutions that may require large antenna coils which are very close (e.g., sometimes within millimeters). Resonant inductive coupling techniques may thus allow for improved efficiency and power transfer over various distances and with a variety of inductive coil configurations.

The receiver 108 may receive power when the receiver 108 is located in the wireless field 105 produced by the transmitter 104. The wireless field 105 corresponds to a region where energy output by the transmitter 104 may be captured by the receiver 108. The wireless field 105 may correspond to the "near-field" of the transmitter 104 as will be further described below. The wireless field 105 may also operate over a longer distance than is considered "near field." The transmitter 104 may include a transmit antenna 114 (e.g., a coil) for transmitting energy to the receiver 108. The receiver 108 may include a receive antenna or coil 118 for receiving or capturing energy transmitted from the transmitter 104. The near-field may correspond to a region in which there are strong reactance fields resulting from the currents and charges in the transmit antenna 114 that minimally radiate power away from the transmit antenna 114. The near-field may correspond to a region that is within about one wavelength (or a fraction thereof) of the transmit antenna 114.

FIG. 2 is a functional block diagram of a wireless power transfer system 200, in accordance with another exemplary implementation. The system 200 includes a transmitter 204 and a receiver 208. The transmitter 204 may include a transmit circuitry 206 that may include an oscillator 222, a driver circuit 224, and a filter and matching circuit 226. The oscillator 222 may be configured to generate a signal at a desired frequency that may be adjusted in response to a frequency control signal 223. The oscillator 222 may provide the oscillator signal to the driver circuit 224. The driver circuit 224 may be configured to drive the transmit antenna 214 at, for example, a resonant frequency of the transmit antenna 214 based on an input voltage signal (V_{D}) 225. The driver circuit 224 may be a switching amplifier configured to receive a square wave from the oscillator 222 and output a sine wave. For example, the driver circuit 224 may be a class E amplifier.

The filter and matching circuit 226 may filter out harmonics or other unwanted frequencies and match the impedance of the transmitter 204 to the impedance of the transmit antenna 214. As a result of driving the transmit antenna 214, the transmit antenna 214 may generate a wireless field 205 to wirelessly output power at a level sufficient for charging a battery 236.

The receiver 208 may include a receive circuitry 210 that may include a matching circuit 232 and a rectifier circuit 234. The matching circuit 232 may match the impedance of the receive circuitry 210 to the receive antenna 218. The rectifier circuit 234 may generate a direct current (DC) power output from an alternate current (AC) power input to charge the battery 236, as shown in FIG. 2. The receiver 208 and the transmitter 204 may additionally communicate on a separate communication channel 219 (e.g., Bluetooth, ZigBee, cellular, etc.). The receiver 208 and the transmitter 204 may alternatively communicate via in-band signaling using characteristics of the wireless field 205.

The receiver 208 may be configured to determine whether an amount of power transmitted by the transmitter 204 and received by the receiver 208 is appropriate for charging the battery 236.

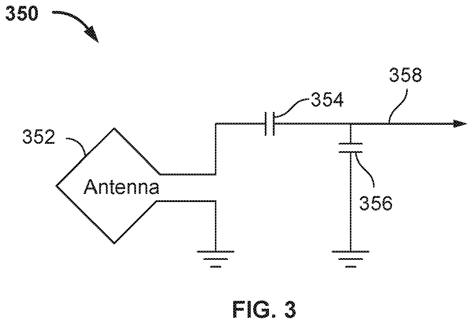

FIG. 3 is a schematic diagram of a portion of the transmit circuitry 206 or the receive circuitry 210 of FIG. 2 including a transmit or receive antenna, in accordance with exemplary implementations. As illustrated in FIG. 3, a transmit or receive circuitry 350 may include an antenna 352. The antenna 352 may also be referred to or be configured as a "loop" antenna 352. The antenna 352 may also be referred to herein or be configured as a "magnetic" antenna or an induction coil. The term "antenna" generally refers to a component that may wirelessly output or receive energy for coupling to another "antenna." The antenna may also be referred to as a coil of a type that is configured to wirelessly output or receive power. As used herein, the antenna 352 is an example of a "power transfer component" of a type that is configured to wirelessly output and/or receive power.

The antenna 352 may include an air core or a physical core such as a ferrite core (not shown).

The transmit or receive circuitry 350 may form/include a resonant circuit. The resonant frequency of the loop or magnetic antennas is based on the inductance and capacitance. Inductance may be simply the inductance created by the antenna 352, whereas, capacitance may be added to the antenna's inductance to create a resonant structure at a desired resonant frequency. As a non-limiting example, a capacitor 354 and a capacitor 356 may be added to the transmit or receive circuitry 350 to create a resonant circuit. For a transmit circuitry, a signal 358 may be an input at a resonant frequency to cause the antenna 352 to generate a wireless field 105/205. For receive circuitry, the signal 358 may be an output to power or charge a load (not shown). For example, the load may comprise a wireless device configured to be charged by power received from the wireless field.

Other resonant circuits formed using other components are also possible. As another non-limiting example, a capacitor may be placed in parallel between the two terminals of the circuitry 350.

Referring to FIGs. 1 and 2, the transmitter 104/204 may output a time varying magnetic (or electromagnetic) field with a frequency corresponding to the resonant frequency of the transmit antenna 114/214. When the receiver 108/208 is within the wireless field 105/205, the time varying magnetic (or electromagnetic) field may induce a current in the receive antenna 118/218. As described above, if the receive antenna 118/218 is configured to resonate at the frequency of the transmit antenna 114/214, energy may be efficiently transferred. The AC signal induced in the receive antenna 118/218 may be rectified as described above to produce a DC signal that may be provided to charge or to power a load.

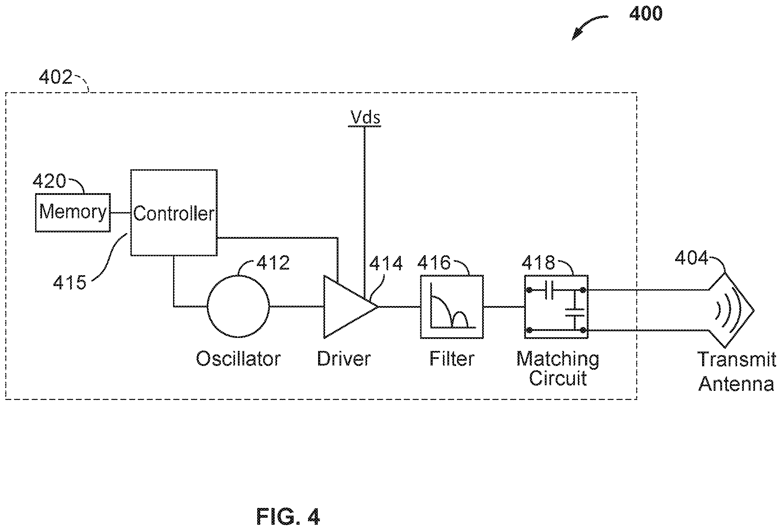

FIG. 4 is a simplified functional block diagram of a transmitter (PTU) that may be used in an inductive power transfer system, in accordance with exemplary implementations of the present disclosure. As shown in FIG. 4, the transmitter or PTU 400 includes transmit circuitry 402 and a transmit antenna 404 operably coupled to the transmit circuitry 402. The transmit antenna 404 may be configured as the transmit antenna 214 as described above in reference to FIG. 2. In some implementations, the transmit antenna 404 may be a coil (e.g., an induction coil). In some implementations, the transmit antenna 404 may be associated with a larger structure, such as a table, mat, lamp, or other stationary configuration. The transmit antenna 404 may be configured to generate an electromagnetic or magnetic field. In an exemplary implementation, the transmit antenna 404 may be configured to transmit power to a receiver device within a charging region at a power level sufficient to charge or power the receiver device.

The transmit circuitry 402 may receive power through a number of power sources (not shown). The transmit circuitry 402 may include various components configured to drive the transmit antenna 404. In some exemplary implementations, the transmit circuitry 402 may be configured to adjust the transmission of wireless power based on the presence and constitution of the receiver devices as described herein. As such, the transmitter 400 may provide wireless power efficiently and safely.

The transmit circuitry 402 may further include a controller 415. In some implementations, the controller 415 may be a micro-controller. In other implementations, the controller 415 may be implemented as an application-specified integrated circuit (ASIC). The controller 415 may be operably connected, directly or indirectly, to each component of the transmit circuitry 402. The controller 415 may be further configured to receive information from each of the components of the transmit circuitry 402 and perform calculations based on the received information. The controller 415 may be configured to generate control signals for each of the components that may adjust the operation of that component. As such, the controller 415 may be configured to adjust the power transfer based on a result of the calculations performed by it.

The transmit circuitry 402 may further include a memory 420 operably connected to the controller 415. The memory 420 may comprise random-access memory (RAM), electrically erasable programmable read only memory (EEPROM), flash memory, or nonvolatile RAM. The memory 420 may be configured to temporarily or permanently store data for use in read and write operations performed by the controller 415. For example, the memory 420 may be configured to store data generated as a result of the calculations of the controller 415. As such, the memory 420 allows the controller 415 to adjust the transmit circuitry 402 based on changes in the data over time.

The transmit circuitry 402 may further include an oscillator 412 operably connected to the controller 415. The oscillator 412 may be configured as the oscillator 222 as described above in reference to FIG. 2. The oscillator 412 may be configured to generate an oscillating signal (e.g., radio frequency (RF) signal) at the operating frequency of the wireless power transfer. In some exemplary implementations, the oscillator 412 may be configured to operate at the 6.78 MHz ISM frequency band. The controller 415 may be configured to selectively enable the oscillator 412 during a transmit phase (or duty cycle). The controller 415 may be further configured to adjust the frequency or a phase of the oscillator 412 which may reduce out-of-band emissions, especially when transitioning from one frequency to another. As described above, the transmit circuitry 402 may be configured to provide an amount of power to the transmit antenna 404, which may generate energy (e.g., magnetic flux) about the transmit antenna 404.

The transmit circuitry 402 may further include a driver circuit 414 operably connected to the controller 415 and the oscillator 412. The driver circuit 414 may be configured as the driver circuit 224 as described above in reference to FIG. 2. The driver circuit 414 may be configured to drive the signals received from the oscillator 412, as described above.

The transmit circuitry 402 may further include a low pass filter (LPF) 416 operably connected to the transmit antenna 404. The low pass filter 416 may be configured as the filter portion of the filter and matching circuit 226 as described above in reference to FIG. 2. In some exemplary implementations, the low pass filter 416 may be configured to receive and filter an analog signal of current and an analog signal of voltage generated by the driver circuit 414. The analog signal of current may comprise a time-varying current signal, while the analog signal of current may comprise a time-varying voltage signal. In some implementations, the low pass filter 416 may alter a phase of the analog signals. The low pass filter 416 may cause the same amount of phase change for both the current and the voltage, canceling out the changes. In some implementations, the controller 415 may be configured to compensate for the phase change caused by the low pass filter 416. The low pass filter 416 may be configured to reduce harmonic emissions to levels that may prevent self-jamming. Other exemplary implementations may include different filter topologies, such as notch filters that attenuate specified frequencies while passing others.

The transmit circuitry 402 may further include a fixed impedance matching circuit 418 operably connected to the low pass filter 416 and the transmit antenna 404. The matching circuit 418 may be configured as the matching portion of the filter and matching circuit 226 as described above in reference to FIG. 2. The matching circuit 418 may be configured to match the impedance of the transmit circuitry 402 (e.g., 50 ohms) to the transmit antenna 404. Other exemplary implementations may include an adaptive impedance match that may be varied based on measurable transmit metrics, such as the measured output power to the transmit antenna 404 or a DC current of the driver circuit 414. The transmit circuitry 402 may further comprise discrete devices, discrete circuits, and/or an integrated assembly of components.

Transmit antenna 404 may be implemented as an antenna strip with the thickness, width and metal type selected to keep resistance losses low.

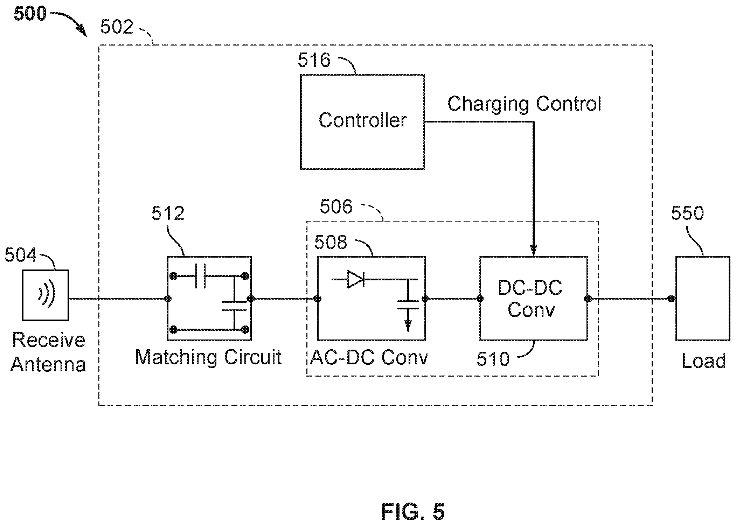

FIG. 5 is a block diagram of a receiver (PRU), in accordance with an implementation of the present disclosure. As shown in FIG. 5, a receiver or PRU 500 includes a receive circuitry 502, a receive antenna 504, and a load 550. The receiver 500 further couples to the load 550 for providing received power thereto. Receiver 500 is illustrated as being external to device acting as the load 550 but may be integrated into load 550. The receive antenna 504 may be operably connected to the receive circuitry 502. The receive antenna 504 may be configured as the receive antenna 218 as described above in reference to FIG. 2. In some implementations, the receive antenna 504 may be tuned to resonate at a frequency similar to a resonant frequency of the transmit antenna 404, or within a specified range of frequencies, as described above. The receive antenna 504 may be similarly dimensioned with transmit antenna 404 or may be differently sized based upon the dimensions of the load 550. The receive antenna 504 may be configured to couple to the magnetic field generated by the transmit antenna 404, as described above, and provide an amount of received energy to the receive circuitry 502 to power or charge the load 550.

The receive circuitry 502 may be operably coupled to the receive antenna 504 and the load 550. The receive circuitry may be configured as the receive circuitry 210 as described above in reference to FIG. 2. The receive circuitry 502 may be configured to match an impedance of the receive antenna 504, which may provide efficient reception of wireless power. The receive circuitry 502 may be configured to generate power based on the energy received from the receive antenna 504. The receive circuitry 502 may be configured to provide the generated power to the load 550. In some implementations, the receiver 500 may be configured to transmit a signal to the transmitter 400 indicating an amount of power received from the transmitter 400.

The receive circuitry 502 may include a processor-signaling controller 516 configured to coordinate the processes of the receiver 500 described below.

The receive circuitry 502 provides an impedance match to the receive antenna 504. The receive circuitry 502 includes power conversion circuitry 506 for converting a received energy into charging power for use by the load 550. The power conversion circuitry 506 includes an AC-to-DC converter 508 coupled to a DC-to-DC converter 510. The AC-to-DC converter 508 rectifies the AC energy signal received at the receive antenna 504 into a non-alternating power while the DC-to-DC converter 510 converts the rectified AC energy signal into an energy potential (e.g., voltage) that is compatible with the load 550. Various AC-to-DC converters are contemplated including partial and full rectifiers, regulators, bridges, doublers, as well as linear and switching converters.

The receive circuitry 502 may further include a matching circuit 512. The matching circuit 512 may comprise one or more resonant capacitors in either a shunt or a series configuration. In some implementations these resonant capacitors may tune the receive antenna to a specific frequency or to a specific frequency range (e.g., a resonant frequency).

The load 550 may be operably connected to the receive circuitry 502. The load 550 may be configured as the battery 236 as described above in reference to FIG. 2. In some implementations the load 550 may be external to the receive circuitry 502. In other implementations the load 550 may be integrated into the receive circuitry 502.

Exposure of a user body to external interference may damage or adversely impact the electronic device implanted or worn on the user body. For example, exposure to X-rays signals and fields, magnetic resonance imaging (MRI) signals and fields, and computed tomography (CT) scan signals and fields may damage the electronic devices and may adversely impact the health condition of the user and/or the functionality of electronic devices themselves. Given the prevalence of such external interference and the increased use of implants and/or other electronic devices for monitoring and controlling human body functions, potential damage to the electronic devices is of growing concern. Accordingly, the electronic devices may be implemented with shielding structures that may prevent the external interference from damaging or otherwise adversely impacting internal components of the electronic device that are located within the shielding structure.

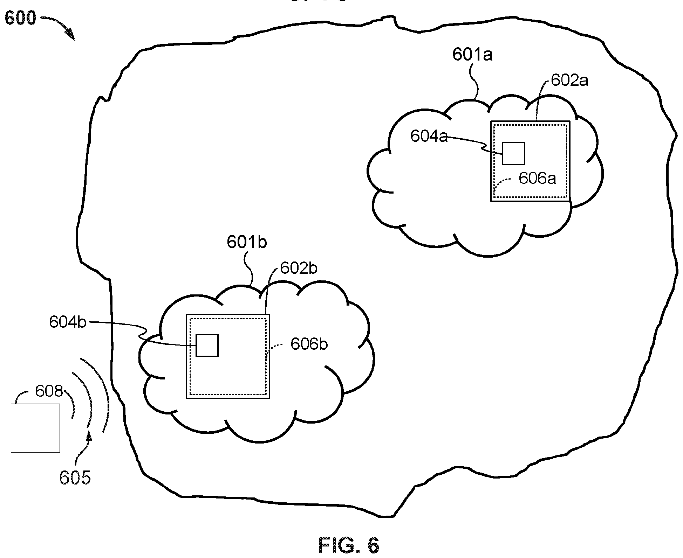

FIG. 6 shows a system 600 of implants located within an area of a human body having two regions or tissues. The system 600 comprises two implants 602a and 602b. Each of the implants 602a and 602b comprises internal circuit components 604a and 604b, respectively. The implants 602a and 602b each further comprise a shield 606a and 606b, respectively, that protects the internal circuit components 604a and 604b from external interference or electrical signals or fields external to the implants 602a and 602b. In some embodiments, the internal circuit components 604a and 604b may each comprise a receiver (not shown) configured to receive power and/or data wirelessly from a transmitter 608 via a wireless field 605. In some embodiments, the respective receivers may be transceivers also configured to transmit power and/or data wirelessly from the implants 602a and 602b. In some embodiments, the receivers correspond to the receiver 500 of FIG. 5. The internal circuit components 604a and 604b of the implants 602a and 602b, respectively, may correspond to the load 550 of FIG. 5 when they receive power via the receivers. The transmitter 608 corresponds to the transmitter 400 of FIG. 4. The area comprises two regions 601a and 601b. The two regions 601a and 601b may each correspond to a different type of tissue within the area of the body. For example, region 601a may correspond to muscle tissue while the region 601b may correspond to bone. The implant 602a is located within the region 601a, while the implant 602b is located within the region 601b.

The area of the body of the system 600 may be replaced by an area of any other living body within which one or more functions may be desired to be monitored or controlled. In the area of the human body as depicted in FIG. 6, the implants 602a and 602b (e.g., comprising various electronic devices) may control or monitor various functions, signals, or conditions of the body.

The implants 602a and 602b may allow for the diagnosis and/or treatment of diseases and/or various other conditions. In some embodiments, the implants 602 may be used for medical "neuromodulation," where the implants 602 attach to nerves of the body and monitor or stimulate the nerves to which they are attached. In some embodiments, the implants 602 may control or regulate a status or a chemical value (e.g., control an introduction of a chemical) of the body. For example, the implants 602 may monitor a brain or nervous system and deliver electrical stimulation or medication to relieve pain and/or restore functions. Alternatively, or additionally, the implants 602 may comprise insulin monitors, insulin injectors, hearing aids, or pacemakers, among other implanted or wearable devices that may be used in relation to various conditions, including Type II Diabetes, rheumatism, and ovary stimulation.

In some embodiments, the implants 602 may utilize primary batteries as a power source. However, as the batteries require replacement, replacement of the batteries in the implants 602 may require surgery to perform the replacement. Accordingly, alternate, or additional, methods of powering the implants 602 are desired. Wireless charging and/or power transfer may provide a safer and less invasive method of powering such implants 602 in the long term. The transmitter 608 may transfer power wirelessly via the wireless field 605 to charge or power the internal circuit components 604a and 604b of such implants 602 via their respective receivers.

However, as described above, the implants 602a and 602b may receive or transmit power and/or data wirelessly via an antenna (not shown). The shielding 606 may impede the wireless transfer of power or data, especially when the antenna used for the transfer is positioned within the shielding 606. Accordingly, in some embodiments, the shielding 606 may be configured to function as the antenna. Such a configuration is described in more detail herein.

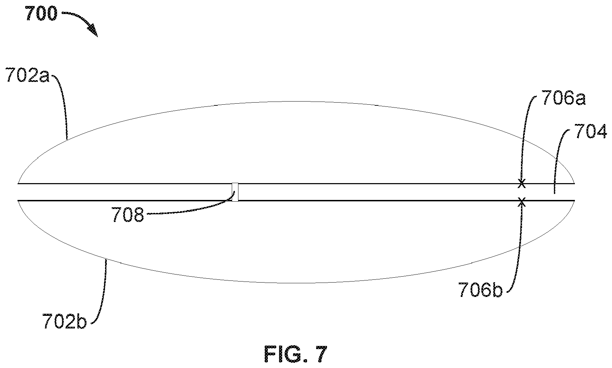

FIG. 7 shows an implant 700 having a shield 702 placed around electrical components within a housing (not shown in this figure). In some embodiments, the shield 702 may be placed outside the housing of the implant 700, where the housing contains internal circuit components (not shown) of the implant 700. In some embodiments, the shield 702 may have a slot 704 that separates the shield 702 into two separate shield pieces 702a and 702b. The two separate shield pieces 702a and 702b of the shield 702 may be connected via a bridge 708 that spans the slot 704. Additionally, each of the two separate pieces 702a and 702b comprises one of the feed connections ("feeds") 706a and 706b. The feeds 706a and 706b may correspond to locations at which a power source or a load (in wireless power transfer context) or a receiver or a transmitter circuit (in data or other information communication) (not shown) may be coupled to the shield pieces 702a and 702b for transmitting and/or receiving the power and/or the data via the shield 702 operating as an antenna. For example, one side of the shield, e.g., shield piece 702a, couples to an output/input of a transceiver/receiver and the other side of the shield, e.g., shield piece 702b, couples to a reference ground on a printed circuit board (PCB). In some implementations, the shield 702 can be made with any conductive metal that is bio-compatible (e.g., does not interfere with or cause a biological reaction inside the user's body). An exemplary metal is titanium.

In some implementations, a common ground of one or more internal circuits may be coupled to one or more of the shield pieces 702a and 702b, as long as a current that flows between the feeds 706a and 706b is not interrupted by the ground. For example, the common ground can be connected to any area inside the shield pieces 702a and 702b that is not close to or along the edges of the pieces that form the slot 704. In some implementations, the common ground may be connected to the bridge 708.

In some embodiments, the housing may be non-conductive and may house the shield 702 and the internal circuit components may be housed by both the shield 702 and the housing. In some embodiments, a thickness of the shield 702 may be dependent at least in part on a penetrating depth into the body of the interference from which the implant 700 is being shielded. In some embodiments, the slot 704 may be replaced by or may comprise an opening, gap, or hole, etc. In some embodiments, the slot 704 may be replaced by or may comprise a plurality of slots, openings, gaps, or holes, etc., that create more than two separate pieces 702a-702x that collectively form the shield 702. For example, the shield 702 may comprise four pieces formed by two slots. In some embodiments, the slot (or slots) 704 may provide a path for various feeds or other connections to couple to circuitry housed within the shield 702. Additionally, or alternatively, the slot (or slots) 704 may be filled with a bio-compatible materials, for example a bio-compatible ceramic, aluminum-zirconia, or a bio-compatible epoxy. In some embodiments, the feeds 706 may be placed at any location, for example, on different pieces of the shield 702, so long as the different pieces of the shield 702 are electrically coupled such that signals may flow between the two shield pieces 702 to the two feeds 706a and 706b.

In some designs, the shield 702 may comprise multiple combinations of feeds 706 along the shield pieces (not shown). In some embodiments, the locations of the combinations of feeds may be dependent upon frequency (e.g., 1 GHz vs. 3 GHz). For example, one or more combinations of feeds may receive or may generate wireless fields at low frequency bands (e.g., 1.6GHz frequencies) via the shield 702. In some embodiments, one or more combinations of feeds may receive or may generate wireless fields at higher frequency bands (e.g., at 2.4GHz frequencies) via the shield 702. A feed location may be determined, at least in part, based on a reference impedance. For example, feed locations for different frequencies may be determined based on the respective frequency and the reference impedance. In some implementations, a single feed location may provide a resonance at a single frequency (e.g., 900MHz) while two feed locations may provide resonances at two frequencies (e.g., 900MHz and 1900MHz). Accordingly, multiple feeds positioned along the slot 704 may support multiple specific frequencies. A ratio of voltage and current (e.g., an impedance) along the slot 704 may determine the locations of the feeds. If the impedance is matched to a design reference impedance (e.g., 50 ohm), then a majority of an excited energy is transmitted through the shield. In other words, no, or reduced, reflection occurs.

In some embodiments, the shield 702 (comprising the plurality of separate pieces as described herein) may be configured to form and operate as an antenna (similar to the antenna 352 of FIG. 2) configured to participate in wireless power and/or data transfer, either as a receive antenna or a transmit antenna. In some embodiments, the shield 702 may be coupled to a source or a load (not shown) via the feeds 706. The source may comprise a transmit circuit, current feed, and/or a power source when the shield 702 is configured to operate as a transmit antenna, or may comprise a receiver and/or a conversion circuitry when the shield 702 is configured to operate as a receive antenna. The shield 702 functioning as the antenna may contain one or more respective bandwidths of transmitting and receiving frequencies. For example, the shield 702 may have a first bandwidth of 925MHz to 960MHz frequencies for receiving power and/or communications and a second bandwidth of 880MHz to 915MHz for transmitting power and/or communications.

The shield 702 and bridge 708 may be configured to substantially form a loop (or "coil antenna") around at least a portion of the implant 700. In some embodiments, the slot 704 may be configured to cause the shield 702 separated into multiple shield pieces, coupled with multiple bridges 708, to form a plurality of loops around at least the portion of the implant 700 and may thus form a multi-loop antenna. When acting as a receive antenna, the shield 702 and bridge 708 may be configured to generate a current in response to being exposed to a field (not shown) and/or receive data transmitted within or via the field. The generated current may be transferred to a receive circuit or load, etc., to which the shield 702 is connected via the feeds 706. When acting as a transmit antenna, the shield 702 and bridge 708 may be configured to generate the field to transmit wireless power and/or transmit data when receiving a current and/or data from a transmit circuit coupled to the shield 702 via the feeds 706. In some embodiments, the shield 702 may be configure to couple to other receive/transmit circuits, for example, NFC circuits, Bluetooth circuits, Wi-Fi circuits, etc. In some embodiments, when the shield 702 is formed from multiple pairs of shield pieces, each pair of shield pieces and its associated bridge may be configured to operate as an antenna for a different transmit/receive circuit to which it is coupled via feeds 706.

By separating the shield 702 into the individual shield pieces 702a and 702b that form the loop via the bridge 708, wireless power and/or data communication may be enabled. The bridge 708 may allow electric currents to flow on and between both of the shield pieces 702a and 702b. In some embodiments, the bridge 708 may serve to allow current to flow between the shield pieces 702a and 702b when the shield 702 is used as a resonator at the determined resonant frequency. When operating as an antenna, then the current across the bridge 708 may be in phase. In some embodiments, the bridge may serve to allow the shield pieces 702a and 702b to serve to cancel current, for example, when the implant 700 is exposed to an interference at a frequency that is not its resonant frequency, e.g., when a current on the shield piece 702a has a different phase than a current on the shield piece 702b. Accordingly, the implant 700 may be configured to attenuate frequencies that are not its resonant frequency based on a configuration of the shield pieces 702 and slot(s) 704. At its resonant frequency, the shield 702 may be configured to operate as a resonator. At other frequencies, the shield 702 may be configured to operate as a shield. Additionally, shield 702 may serve to mitigate field penetration by providing an additional layer through which the field must penetrate. In some implementations, a thickness of the shield 702 may mitigate field penetration of the shield 702. For example, the thickness of the shield 702 may be large enough to prevent direct penetration of a possible magnetic field. Accordingly, the shield thickness may be several times thicker than the skin depth penetration of an incoming interference frequency.

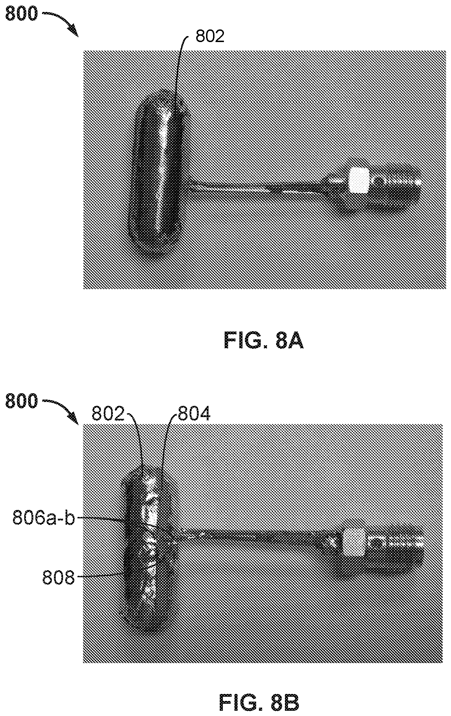

FIG. 8A shows a first view of an implant 800 having a shield and a coaxial (e.g., multiple conductor) feed. The implant 800 is shown having a pill-like oblong shape. The exterior of the implant is covered or surrounded by the shield 802. In some embodiments, the shield may be made of ferromagnetic metal or some other magnetic material (to block incoming magnetic fields) or other conductive materials (to block other incoming electric fields).

FIG. 8B shows a second view of the implant 800 of FIG. 8A. The implant 800 comprises the shield 802. The shield 802 has a slot 804 that divides the shield into two separate pieces. Through the slot 804, a housing containing internal electronic components of the implant 800 is visible. Additionally, feeds 806a and 806b are shown on opposite sides of the slot 804 on the separate pieces of the shield 802. One feed 806a may be connected to the left piece of the shield 702a while the other feed 806b is connected to the right piece of the shield 702b. The bridge 808 is shown coupling the two pieces of the shield 802. In some embodiments, the bridge 808 may be switchable or movable based on a desired frequency of attenuation to enable functionality at different selectable frequencies.

When the implant 800 is exposed to a field, the shield 802 may resonate and generate a voltage which is applied to the feeds 806a and 806b to a receive circuit and/or load. When configured to transmit power and/or data, the shield 802 of the implant 800 may be coupled to a power source via the feeds 806a and 806b and may generate a field used to transmit the power and/or the data to a receiver within the field.

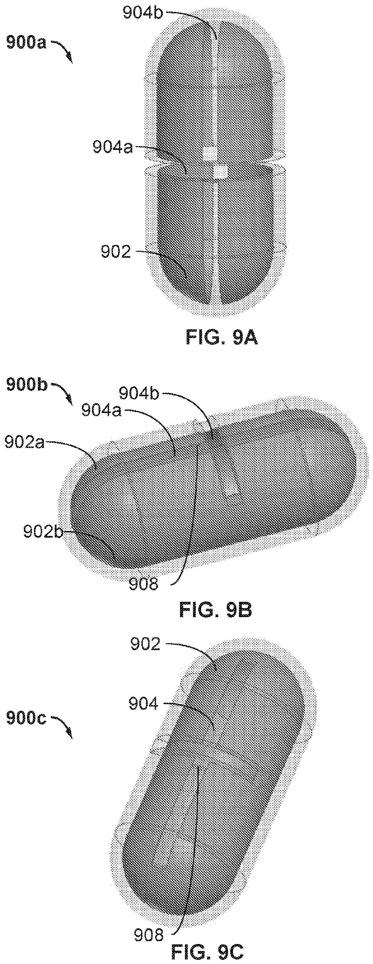

FIGs. 9A-9C show alternate configurations of the shield, slot, and/or bridge of the implant of FIGs. 7 and 8. For example, FIG. 9A shows two perpendicular slots 904a and 904b that separate the shield 902 into four separate shield pieces 902a-902d. Though not shown, multiple bridges may be used to connect to the shield pieces 904a-904d in various configurations to maximize slot length, which, accordingly, determines a resonance frequency of the shield 902. Such a configuration of slots 904a and 904b and shield pieces 902a-902d may provide for lower resonant frequency responsiveness and may simplify fabrication of the shield 902. In some embodiments, the configuration of slots 904 and shield pieces 902 may enable the implant 900 to have any resonant frequency, where different combinations of slots 904, bridges 908, and shield pieces 902 may enable different frequency attenuation configurations.

FIG. 9B shows two perpendicular slots 904a and 904b, where the slot 904a does travel along a circumference of the shield 902 and separates the shield 902 into two distinct portions, 902a and 902b. However, the slot 904b does not travel a circumference of the shield 902 and, thus, does not divide the shield into distinct pieces. The configuration of the slots 904a and 904b (e.g., the slot 904b may be referred to as a shorted slot) may serve to increase a length of the slot, and currents may follow the slots 904a and 904b and may provide for equivalently increased antenna geometries. Accordingly, the induced current may flow along the slot, which allows for a smaller resonant frequency than the embodiment shown in FIGs. 7A-7B. A bridge 908 is shown spanning the slot 904a.

The shield 902 of FIG. 9A may provide a lower resonant frequency as compared to the shield 902 of FIG. 9B. However, the shield 902 of FIG. 9B may provide better shielding than the shield 902 of FIG. 9A. For example, as the slot 904 of FIG. 9B is smaller than that of slot 904 of FIG. 9A, the shield 902 of FIG. 9B may provide better shielding than the shield 902 of FIG. 9A.

FIG. 9C shows a single shorted slot 904 that does not separate the shield 902 into separate pieces. A bridge 908 is also shown spanning the slot 904. In such an embodiment, the slot 904 may be the radiating origin when the shield 902 is used to transmit. The positioning of the slot 904 and the bridge 908 may allow for a more directional radiation from the slot antenna formed by the shield 902 and the slot 904. Thus, specifying the location of the slot 904 may allow for the establishing of direction of transmission and/or reception by the implant using the shield 902 having the slot 904 as the antenna of the implant. In some embodiments, the bridge 908 may not be necessary as the shield 902 is not separated into separate pieces.

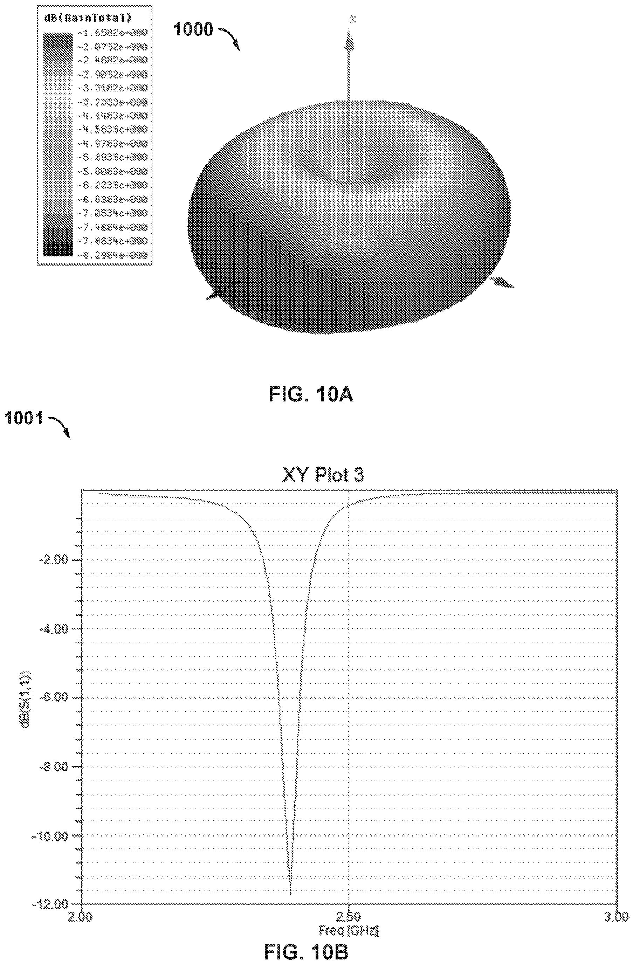

FIG. 10A shows a 3D graph 1000 corresponding to a radiation pattern of the antenna of the implant (e.g., the implant 700 of FIG. 7). The graph comprises an x-axis, a y-axis, and a z-axis. The x-axis corresponds to the length of the implant 700 (e.g., a primary direction of the longest slot of the implant), while the y- and z-axes correspond with a width and height of the implant. The x-axis shows a null, while the y- and z-axes show an omnidirectional radiation pattern. Thus, when the implant 700 is positioned within the area of the body such that the length (e.g., the longest dimension of the implant) of the implant is along a longest dimension of the body within an abdomen of the body, the radiation pattern of the implant (shown in the y- and z-axes) may be oriented around a waist of the body, providing omni-directional wireless connectivity.

FIG. 10B shows a graph 1001 indicating signal strength of the antenna of the implant (e.g., the implant 700 of FIG. 7) as a function of frequency. The graph 1001 comprises an x-axis and a y-axis. The x-axis shows frequencies, while the y-axis shows a reflection coefficient. The reflection coefficient may indicate how much of incident power is reflected by the implant. As shown, the reflection coefficient is -12dB at 2.4 GHz, which means most of input signal is coming into the antenna. Accordingly, the resonant frequency of the antenna of the implant is approximately 2.4 GHz and that the antenna radiates most efficiently at that frequency.

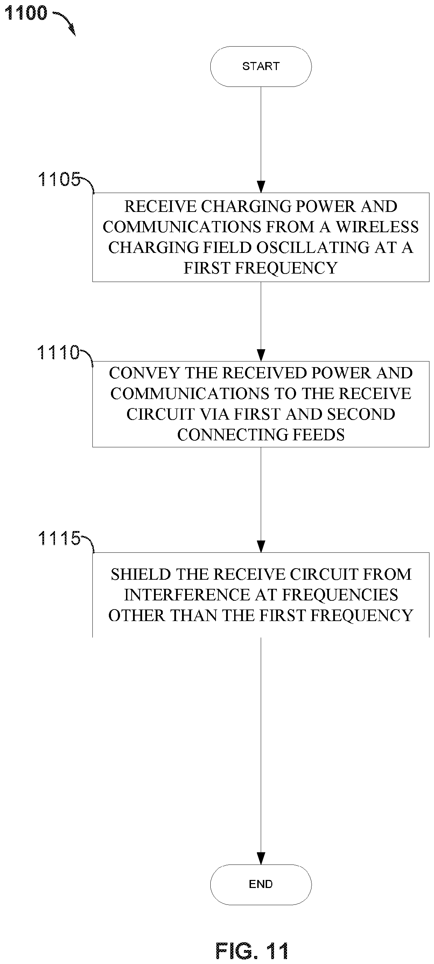

FIG. 11 is a flowchart that includes a plurality of steps of a method 1100 for receiving and/or transmitting wireless power and communication in an implantable device, in accordance with exemplary implementations of the present disclosure. For example, the method 1100 could be performed by the PRU 500 illustrated in FIG. 5. Method 1100 may also be performed by the implants 700 of FIG. 7. A person having ordinary skill in the art will appreciate that the method 1100 may be implemented by other suitable devices and systems. Although the method 1100 is described herein with reference to a particular order, in various implementations, blocks herein may be performed in a different order, or omitted, and additional blocks may be added.

The method 1100 begins at operation block 1105 with the implant 700 receiving charging power and communications from a wireless charging field oscillating at a first frequency, the wireless charging field generated by a power transmitter unit (PTU). Specifically, the shield 702 of the implant 700 may physically receive the charging power and communications from the wireless charging field. In some implementations, the shield 702 may resonate in response to being exposed to the wireless charging field.

At operation block 1110, the implant 700 (e.g., via the shield 702) conveys the received charging power and communications to a receive circuit housed within the shield 702 via first and second connecting feeds. In some implementations, the implant 700 may receive only one of charging power or communications via the shield 702. In some implementations, the received power may be used to power the receive circuit or other circuits or circuitry housed within the shield 702.

At operation block 1115, the shield 702 of the implant 700 shields the receive circuit from interference at frequencies other than the first frequency. Accordingly, the shield 702 may be configured to attenuate frequencies other than the first frequency. In some implementations, shield 702, though described in relation to receiving power and communications, may be configured to transmit one or more of power and communications while shielding a transmit circuit housed within the shield 702 from interference and frequencies other than a designed transmit frequency.

An apparatus for wirelessly receiving power may perform one or more of the functions of method 1100, in accordance with certain implementations described herein. The apparatus may comprise means for receiving charging power and communications from a wireless charging field oscillating at a first frequency. In certain implementations, the means for receiving charging power and communications can be implemented by the shield 702 (FIG. 7), the shield 802 (FIG. 8), or the shield 902 (FIG. 9). In some implementations, the means for receiving charging power and communications can be implemented by the implant 700, the implant 800, or the implant 900. In certain implementations, the means for receiving charging power and communications can be configured to perform the functions of block 1105 (FIG. 11). The apparatus may further comprise means for conveying the received power and communications to a receive circuit via first and second connecting feeds. In certain implementations, the means for conveying the received power and communications can be implemented by one or more feeds connected to the shield 702 at feed connections or feeds 706a and 706b (FIG. 7) or feeds 806a and 806b (FIG. 8). In certain implementations, the means for conveying the received power and communications can be configured to perform the functions of block 1110 (FIG. 11).

The apparatus may further comprise means for shielding the receive circuit from interference at frequencies other than the first frequency. In certain implementations, the means for shielding the receive circuit can be implemented by the shield 702, 802, or 902. In certain implementations, the means for shielding the receive circuit can be configured to perform the functions of block 1115 (FIG. 11).

In some embodiments, an apparatus for receiving charging power and communications may comprise, in some implementations, the PRU 500 of FIG. 5 (specifically the antenna 504 that is configured to house the receive circuitry 502) and the PRU 500 may perform associated functions and methods described herein.

The various operations of methods described above may be performed by any suitable means capable of performing the operations, such as various hardware and/or software component(s), circuits, and/or module(s). Generally, any operations illustrated in the Figures may be performed by corresponding functional means capable of performing the operations.

Information and signals may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

The various illustrative logical blocks, modules, circuits, and method steps described in connection with the implementations disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. The described functionality may be implemented in varying ways for each particular application, but such implementation decisions should not be interpreted as causing a departure from the scope of the implementations.

The various illustrative blocks, modules, and circuits described in connection with the implementations disclosed herein may be implemented or performed with a general purpose hardware processor, a Digital Signal Processor (DSP), an Application Specified Integrated Circuit (ASIC), a Field Programmable Gate Array (FPGA) or other programmable logic device, discrete gate or transistor logic, discrete hardware components, or any combination thereof designed to perform the functions described herein. A general purpose hardware processor may be a microprocessor, but in the alternative, the hardware processor may be any conventional processor, controller, microcontroller, or state machine. A hardware processor may also be implemented as a combination of computing devices, e.g., a combination of a DSP and a microprocessor, a plurality of microprocessors, one or more microprocessors in conjunction with a DSP core, or any other such configuration.

The steps of a method and functions described in connection with the implementations disclosed herein may be embodied directly in hardware, in a software module executed by a hardware processor, or in a combination of the two. If implemented in software, the functions may be stored on or transmitted as one or more instructions or code on a tangible, non-transitory computer readable medium. A software module may reside in Random Access Memory (RAM), flash memory, Read Only Memory (ROM), Electrically Programmable ROM (EPROM), Electrically Erasable Programmable ROM (EEPROM), registers, hard disk, a removable disk, a CD ROM, or any other form of storage medium known in the art. A storage medium is coupled to the hardware processor such that the hardware processor can read information from, and write information to, the storage medium. In the alternative, the storage medium may be integral to the hardware processor. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer readable media. The hardware processor and the storage medium may reside in an ASIC.

For purposes of summarizing the disclosure, certain aspects, advantages and novel features have been described herein. It is to be understood that not necessarily all such advantages may be achieved in accordance with any particular implementation. Thus, the present disclosure may be embodied or carried out in a manner that achieves or optimizes one advantage or group of advantages as taught herein without necessarily achieving other advantages as may be taught or suggested herein.

## Claims

1. An apparatus for receiving wireless power and communications, comprising:
means for receiving (902) wireless charging power and communications from a wireless charging field oscillating at a first frequency via a metallic structure, the metallic structure defining a slot (904) extending through a first surface and a second surface of the metallic structure ;
means for conveying (902) the received power and communication to a receive circuit (502); and
the metallic structure further comprising a plurality of shield pieces and configured to shield the receive circuit (502) from interference at frequencies other than the first frequency via configuration of the slot and shield pieces.

2. The apparatus of claim 1, further comprising a metal bridge (908) that connects a first portion (902a) of the means for receiving and a second portion (902b) of the means for receiving, wherein the slot (904) divides the means for receiving into the first and second portions.

3. The apparatus of claim 1, wherein the means for receiving further comprises a housing that houses the receive circuit.

4. The apparatus of claim 3, wherein the receive circuit comprises one or more components of an implantable device implanted within a body, further comprising means for conveying the power and communications to the implantable device.

5. The apparatus of claim 1, further comprising a first metal bridge (908), a second metal bridge (908), and a third metal bridge (908), wherein the slot (904) divides the means for receiving into a first portion (902a), a second portion (902b), a third portion (902c), and a fourth portion (902d) and wherein the first metal bridge, second, and third metal bridges (908) are configured to connect the first, second, third, and fourth pieces to maximize a length of the slot (904).

6. The apparatus of claim 1, further comprising means for inducing currents on the means for receiving that follow a first slot and a second slot orthogonal and connected to the first slot, wherein the first slot and the second slot form the slot (904).

7. The apparatus of claim 6, wherein one or more of the first and second slots (904a, 904b) is filled with a bio-compatible material and wherein the bio-compatible material is one of ceramic, aluminum-zirconia, and epoxy.

8. The apparatus of claim 6, wherein one or more of the first and second slots (904a, 904b) provides a pathway for connections from one or more circuits housed within the means for receiving to sensors or devices outside the means for receiving.

9. The apparatus of claim 1, further comprising a transmit circuit (402) configured to transmit wireless communication via the metallic structure (902) and wherein the metallic structure (902) transmits the wireless communication as received from the transmit circuit (402) to another device.

10. The apparatus of claim 1, wherein the metallic structure (902) is further configured to receive the communication from the wireless charging field oscillating at a first frequency.

11. A method (1100) of receiving wireless power and communications, comprising:
receive (1105) wireless charging power and communications from a wireless charging field oscillating at a first frequency via a metallic structure, the metallic structure (902) defining a slot (904) extending from a first surface of the metallic structure (902) to a second surface of the metallic structure, and through the metallic structure, the first surface opposite the second surface;
convey (1110) the received power and communication to a receive circuit via first and second connecting wires; and
shielding (1115), via configuration of the slot (904) and a plurality of shield pieces of the metallic structure, the receive circuit from interference at frequencies other than the first frequency.

12. The method of claim 11, further comprising inducing currents on the metallic structure (902) that follow a first slot (904a) and a second slot (904b) orthogonal and connected to the first slot (904a), wherein the first slot and the second slot form the slot (904).

13. The method of claim 11, further comprising transmitting wireless communication via the metallic structure (902), wherein the metallic structure (902) transmits the wireless communication as received from a transmit circuit (402) to another device.

## Patentansprüche

1. Eine Vorrichtung zum Empfangen von Drahtlosleistung und -kommunikationen, die Folgendes aufweist:
Mittel zum Empfangen (902) von Drahtlosladeleistung und -kommunikationen von einem Drahtlosladefeld, das mit einer ersten Frequenz oszilliert, über eine Metallstruktur, wobei die Metallstruktur einen Schlitz (904) definiert, der sich durch eine erste Oberfläche und eine zweite Oberfläche der Metallstruktur erstreckt;
Mittel zum Übermitteln (902) der empfangenen Leistung und Kommunikation an eine Empfangsschaltung (502); und
wobei die Metallstruktur weiter eine Vielzahl von Abschirmungsstücken aufweist und konfiguriert ist zum Abschirmen der Empfangsschaltung (502) gegenüber Interferenz bei anderen Frequenzen als der ersten Frequenz mittels Konfiguration des Schlitzes und der Abschirmungsstücke.

2. Vorrichtung nach Anspruch 1, die weiter eine Metallbrücke (908) aufweist, die einen ersten Teil (902a) der Mittel zum Empfangen und einen zweiten Teil (902b) der Mittel zum Empfangen verbindet, wobei der Schlitz (104) die Mittel zum Empfangen in den ersten und zweiten Teil teilt.

3. Vorrichtung nach Anspruch 1, wobei die Mittel zum Empfangen weiter ein Gehäuse aufweisen, das die Empfangsschaltung beherbergt.

4. Vorrichtung nach Anspruch 3, wobei die Empfangsschaltung eine oder mehrere Komponenten einer implantierbaren Einrichtung aufweist, die in einen Körper implantiert ist, die weiter Mittel zum Übermitteln der Leistung und Kommunikationen an die implantierbare Einrichtung aufweist.

5. Vorrichtung nach Anspruch 1, die weiter eine erste Metallbrücke (908), eine zweite Metallbrücke (908) und eine dritte Metallbrücke (908) aufweist, wobei der Schlitz (904) die Mittel zum Empfangen in einen ersten Teil (902a), einen zweiten Teil (902b), eine dritten Teil (902c) und einen vierten Teil (902d) teilt, und wobei die erste Metallbrücke, zweite Metallbrücke und dritte Metallbrücke (908) konfiguriert sind zum Verbinden des ersten, zweiten, dritten und vierten Teils zum Maximieren einer Länge des Schlitzes (904).

6. Vorrichtung nach Anspruch 1, die weiter Mittel aufweist zum Induzieren von Strömen auf den Mitteln zum Empfangen, die einem ersten Schlitz und einem zweiten Schlitz, der zu dem ersten Schlitz orthogonal ist und mit diesem verbunden ist, folgen, wobei der erste Schlitz der zweite Schlitz den Schlitz (904) bilden.

7. Vorrichtung nach Anspruch 6, wobei einer oder mehrere von dem ersten und zweiten Schlitz (904a, 904b) mit einem biokompatiblen Material gefüllt sind und wobei das biokompatible Material eines von Keramik, Aluminium-Zirkoniumdioxid und Epoxid ist.

8. Vorrichtung nach Anspruch 6, wobei eine oder mehrere von den ersten und zweiten Schlitzen (904a, 904b) einen Pfad für Verbindungen von einer oder mehreren Schaltungen vorsieht, die innerhalb der Mittel zum Empfangen beherbergt werden, zu Sensoren oder Einrichtungen außerhalb der Mittel zum Empfangen.

9. Vorrichtung nach Anspruch 1, die weiter eine Sendeschaltung (402) aufweist, die konfiguriert ist zum Senden von Drahtloskommunikation über die Metallstruktur (902) und wobei die Metallstruktur (902) die Drahtloskommunikation, wie sie von der Sendeschaltung (402) empfangen wird, an eine andere Einrichtung sendet.

10. Vorrichtung nach Anspruch 1, wobei die Metallstruktur 9102) weiter konfiguriert ist zum Empfangen der Kommunikation von dem Drahtlosladefeld, das mit einer ersten Frequenz oszilliert.

11. Ein Verfahren (1100) zum Empfangen von Drahtlosleistung und -kommunikationen, das Folgendes aufweist:
Empfangen (1105) von Drahtlosladeleistung und -kommunikationen von einem Drahtlosladefeld, das mit einer ersten Frequenz oszilliert, über eine Metallstruktur, wobei die Metallstruktur (902) einen Schlitz (904) definiert, der sich von einer ersten Oberfläche der Metallstruktur (902) zu einer zweiten Oberfläche der Metallstruktur und durch die Metallstruktur erstreckt, wobei die erste Oberfläche der zweiten Oberfläche gegenüberliegt;
Übermitteln (1110) der empfangenen Leistung und Kommunikation an eine Empfangsschaltung über erste und zweite Verbindungsdrähte; und
Abschirmen (1115), mittels Konfiguration des Schlitzes (904) und einer Vielzahl von Abschirmungsstücken der Metallstruktur, der Empfangsschaltung gegenüber Interferenz bei anderen Frequenzen als der ersten Frequenz.

12. Verfahren nach Anspruch 11, das weiter Induzieren von Strömen auf der ersten Metallstruktur (902), die einem ersten Schlitz (904a) und einem zweiten Schlitz (904b), der zu dem ersten Schlitz (904a) orthogonal und mit diesem verbunden ist, folgen, wobei der erste Schlitz und der zweite Schlitz den Schlitz (904) bilden.

13. Verfahren nach Anspruch 11, das weiter Senden von Drahtloskommunikation über die Metallstruktur (902) aufweist, wobei die Metallstruktur (902) die Drahtloskommunikation, wie sie von einer Sendeschaltung (402) empfangen wird, an eine andere Einrichtung sendet.

## Revendications

1. Appareil pour recevoir de l'énergie ainsi que des communications sans fil, comprenant :
des moyens de réception (902) de l'énergie de charge et des communications sans fil provenant d'un champ de charge sans fil oscillant à une première fréquence via une structure métallique, la structure métallique définissant une fente (904) s'étendant à travers une première surface et une deuxième surface de la structure métallique ;
des moyens pour acheminer (902) l'énergie et la communication reçues à un circuit de réception (502) ; et
la structure métallique comprenant en outre une pluralité de pièces de blindage et étant configurée pour protéger le circuit de réception (502) des interférences à des fréquences autres que la première fréquence via la configuration de la fente et des pièces de blindage.

2. Appareil selon la revendication 1, comprenant en outre un pont métallique (908) qui relie une première partie (902a) des moyens de réception et une deuxième partie (902b) des moyens de réception, dans lequel la fente (904) divise les moyens de réception dans les première et deuxième parties.

3. Appareil selon la revendication 1, dans lequel les moyens de réception comprennent en outre un boîtier qui abrite le circuit de réception.

4. Appareil selon la revendication 3, dans lequel le circuit de réception comprend un ou plusieurs composants d'un dispositif implantable implanté dans un corps, comprenant en outre des moyens pour transmettre l'énergie et les communications au dispositif implantable.

5. Appareil selon la revendication 1, comprenant en outre un premier pont métallique (908), un deuxième pont métallique (908) et un troisième pont métallique (908), dans lequel la fente (904) divise les moyens de réception en une première partie (902a), une deuxième partie (902b), une troisième partie (902c) et une quatrième partie (902d) et dans lequel le premier pont métallique, les deuxième et troisième ponts métalliques (908) sont configurés pour connecter les premier, deuxième, troisième et quatrième pièces pour maximiser la longueur de la fente (904).

6. Appareil selon la revendication 1, comprenant en outre des moyens pour induire des courants au niveau des moyens de réception qui suivent une première fente et une deuxième fente orthogonale et connectée à la première fente, dans lequel la première fente et la deuxième fente forment la fente (904).

7. Appareil selon la revendication 6, dans lequel une ou plusieurs des première et deuxième fentes (904a, 904b) sont remplies d'un matériau biocompatible et dans lequel le matériau biocompatible est l'un parmi la céramique, l'aluminium-zircone et l'époxy.

8. Appareil selon la revendication 6, dans lequel une ou plusieurs des première et deuxième fentes (904a, 904b) fournissent une voie pour les connexions depuis un ou plusieurs circuits logés à l'intérieur des moyens de réception vers des capteurs ou des dispositifs à l'extérieur des moyens de réception.

9. Appareil selon la revendication 1, comprenant en outre un circuit de transmission (402) configuré pour transmettre une communication sans fil via la structure métallique (902) et dans lequel la structure métallique (902) transmet la communication sans fil telle qu'elle est reçue du circuit de transmission (402) à un autre dispositif.

10. Appareil selon la revendication 1, dans lequel la structure métallique (902) est en outre configurée pour recevoir la communication du champ de charge sans fil oscillant à une première fréquence.

11. Procédé (1100) pour recevoir de l'énergie ainsi que des communications sans fil, comprenant les étapes consistant à :
recevoir (1105) de l'énergie de charge et des communications sans fil d'un champ de charge sans fil oscillant à une première fréquence via une structure métallique, la structure métallique (902) définissant une fente (904) s'étendant d'une première surface de la structure métallique (902) à une deuxième surface de la structure métallique, et à travers la structure métallique, la première surface opposée à la deuxième surface ;
acheminer (1110) l'énergie et la communication reçues vers un circuit de réception via des premier et deuxième fils de connexion ; et
assurer le blindage (1115), via la configuration de la fente (904) et d'une pluralité de pièces de blindage de la structure métallique, du circuit de réception contre les interférences à des fréquences autres que la première fréquence.

12. Procédé selon la revendication 11, comprenant en outre l'induction de courants sur la structure métallique (902) qui suivent une première fente (904a) et une deuxième fente (904b) orthogonale et connectée à la première fente (904a), dans lequel la première fente et la deuxième fente forme la fente (904).

13. Procédé selon la revendication 11, comprenant en outre la transmission d'une communication sans fil via la structure métallique (902), dans lequel la structure métallique (902) transmet la communication sans fil telle qu'elle est reçue d'un circuit de transmission (402) à un autre dispositif.
